# EUROPÄISCHE PATENTSCHRIFT

(11) **EP 1 340 087 B1**
(45) Veröffentlichungstag und Bekanntmachung des Hinweises auf die Patenterteilung: **30.05.2012**
(21) Anmeldenummer: 01998832.8
(22) Anmeldetag: 27.11.2001
(51) Int. Cl.: G01N 33/569, G01N 33/53, G01N 33/531, G01N 33/543

(54) **VERWENDUNG INTRAMOLEKULAR KOVALENT VERNETZTER PROTEINE ALS BINDEPARTNER IN IMMUNOASSAYS**
USE OF INTRAMOLECULARLY, COVALENTLY CROSS-LINKED PROTEINS AS BINDING PARTNERS IN IMMUNOASSAYS
PROTEINES A RETICULATION COVALENTE INTRAMOLECULAIRES UTILISEES COMME PARTIES LIANTES DANS DES IMMUNO-ESSAIS

(30) Priorität: 30.11.2000 DE 10059720
(43) Veröffentlichungstag der Anmeldung: 03.09.2003
(73) Patentinhaber: Roche Diagnostics GmbH, 68305 Mannheim (DE); F.Hoffmann-La Roche AG, 4070 Basel (CH)
(72) Erfinder: UPMEIER, Barbara, 82393 Iffeldorf (DE); SCHLIEPER, Dittmar, 82393 Iffeldorf (DE); DONIE, Frederic, 82377 Penzberg (DE)
(86) Internationale Anmeldenummer: PCT/EP2001/013780
(87) Internationale Veröffentlichungsnummer: WO 2002/044731

(56) Entgegenhaltungen:
- DE-A- 2 615 349
- US-A- 5 258 501
- DEBYSER ZEGER ET AL: "Chemical crosslinking of the subunits of HIV-1 reverse transcriptase." PROTEIN SCIENCE, Bd. 5, Nr. 2, 1996, Seiten 278-286, XP009000136 ISSN: 0961-8368
- MUELLER B ET AL: "CO-EXPRESSION OF THE SUBUNITS OF THE HETERODIMER OF HIV-1 REVERSE TRANSCRIPTASE IN ESCHERICHIA COLI" JOURNAL OF BIOLOGICAL CHEMISTRY, AMERICAN SOCIETY OF BIOLOGICAL CHEMISTS, BALTIMORE, MD, US, Bd. 264, Nr. 24, 25. August 1989 (1989-08-25), Seiten 13975-13978, XP001037290 ISSN: 0021-9258
- ISHIKAWA SETSUKO ET AL: "Whole saliva dried on filter paper for diagnosis of HIV-1 infection by detection of antibody IgG to HIV-1 with ultrasensitive enzyme immunoassay using recombinant reverse transcriptase as antigen." JOURNAL OF CLINICAL LABORATORY ANALYSIS, Bd. 10, Nr. 1, 1996, Seiten 35-41, XP009000109 ISSN: 0887-8013
- JANIS KUBY: "IMMUNOLOGY" 1994 , W.H. FREEMAN & COMPANY , NEW YORK XP002213873 ISBN: 0-7167-2643-2 S. 113, Fig. 5-4
- KUO K-W ET AL: "IMMUNOCHEMICAL CHARACTERIZATION OF ALPHA BUNGAROTOXIN DETOXIFIED WITH GLUTARALDEHYDE" KAOHSIUNG JOURNAL OF MEDICAL SCIENCES, Bd. 6, Nr. 8, 1990, Seiten 408-417, XP009000124 ISSN: 0257-5655
- INTERNET CITATION, [Online] Gefunden im Internet: <URL:http//www.hepatitisbviruspage.com/hbco re.htm>
- ZHOU SILIANG ET AL: "Hepatitis B virus capsid particles are assembled from core-protein dimer precursors", PROCEEDINGS OF THE NATIONAL ACADEMY OF SCIENCES OF THE UNITED STATES OF AMERICA, vol. 89, no. 21, 1992, pages 10046-10050, ISSN: 0027-8424
- DATABASE BIOSIS [Online] BIOSCIENCES INFORMATION SERVICE, PHILADELPHIA, PA, US September 1998 OU WU ET AL: 'The influence of HCV N-terminal deletion on the molecular interactions between NS3/NS3 and NS3-NS4A' Database accession no. PREV199800509195
- TORCHILIN V.P. ET AL: 'STABILIZATION OF SUBUNIT ENZYMES BY INTRAMOLECULAR CROSSLINKING WITH BIFUNCTIONAL REAGENTS' LASKIN, A. I., G. T. TSAO AND L. B. WINGARD, JR. (ED.). ANNALS OF THE NEW YORK ACADEMY OF SCIENCES, VOL. 434. ENZYME ENGINEERING; 7TH INTERNATIONAL CONFERENCE, WHITE HAVEN, PA., USA, SEPT. 25-30, 1983. XVII+596P. NEW YORK ACADEMY OF SCIENCES: NEW YOR 1984, Seiten 27 - 30 ISSN: null
- FERNANDEZ-LAFUENTE ET AL: "Stabilization of multimeric enzymes: Strategies to prevent subunit dissociation", ENZYME AND MICROBIAL TECHNOLOGY, STONEHAM, MA, US, vol. 45, no. 6-7, 1 December 2009 (2009-12-01), pages 405-418, XP026676922, ISSN: 0141-0229, DOI: 10.1016/J.ENZMICTEC.2009.08.009 [retrieved on 2009-08-25]
- TORCHILIN V.P. ET AL: 'Stabilization of Subunit Enzymes by Intersubunit Crosslinking with Bifunctional Reagents: Studies with Glyceraldehyde-3-Dehydrogenase' JOURNAL OF MOLECULAR CATALYSIS Bd. 19, 1983, Seiten 291 - 301

## Beschreibung

Die vorliegende Erfindung betrifft die Verwendung kovalent vernetzter Reverser Transkriptase von HIV als immunologische Bindepartner in Immunoassays, immunologische Testverfahren zum Nachweis eines Analyten in einer Probe, in dem intramolekular kovalent vernetzte reverse Transkriptase von HIV als Bindepartner eingesetzt werden, intramolekular kovalent vernetzte Reverse Transkriptase von HIV, sowie ein Verfahren zur Herstellung dieser Reversen Transkriptase.

Die Verwendung von Proteinen als Bindepartner für den Nachweis von Analyten in immundiagnostischen Testverfahren ist seit langem bekannt. In allen gängigen Immunoassays wird die Probe mit einem oder mehreren, für den Analyten spezifischen Bindepartnern inkubiert. Der oder die Bindepartner binden spezifisch an den nachzuweisenden Analyten. Im Falle eines Antikörper-Nachweises - beispielsweise bei einer HCV-Infektion - wird die zu untersuchende Probe beispielsweise mit einem HCV-Antigen in Kontakt gebracht, das den nachzuweisenden Anti-HCV-Antikörper spezifisch bindet. Bei einem Antigen-Nachweis wie beispielsweise beim Nachweis des Tumormarkers prostataspezifisches Antigen (PSA) wird die Probe mit Antikörpern in Kontakt gebracht, die spezifisch das PSA in der Probe binden.

Anschließend findet in allen Immunoassays der Nachweis des Analyten statt. Dies kann beispielsweise durch die Bindung und anschließende Detektion eines weiteren, mit einer nachweisbaren Markierung versehenen Bindepartners erfolgen, der an den Komplex aus Analyt und immunologischem Bindepartner bindet.

Im allgemeinen werden die Immunoassays im heterogenen oder homogenen Testformat durchgeführt. Die heterogenen Testformate werden häufig als Sandwich- oder Brückentest durchgeführt. Auch kompetitive Verfahren, bei denen entweder der Analyt oder der spezifische Bindepartner aus dem Komplex aus Analyt und spezifischem Bindepartner beispielsweise durch Zugabe eines markierten Analyt-Analogons verdrängt werden, sind allgemein bekannt.

Wichtig ist bei allen immunologischen Testverfahren, dass die als spezifische immunologische Bindepartner eingesetzten Reaktanten in stabiler Form vorliegen und dass diese beispielsweise aufgrund ungünstiger Lagerbedingungen nicht zerstört werden. Diese Gefahr besteht insbesondere dann, wenn die als spezifische Bindepartner eingesetzten Proteine aus mehreren Untereinheiten zusammengesetzt sind. Die Untereinheiten können kovalent wie zum Beispiel über Disulfidbrücken oder nicht kovalent, beispielsweise über Wasserstoffbrücken, entgegengesetzte Ladungen und/oder hydrophobe Wechselwirkungen zusammengehalten werden.

In einigen Fällen kommt es unter den Lagerbedingungen der für den immunologischen Test benötigten Einsatzstoffe (beispielsweise als Flüssigreagenz), in den für den Test hergestellten Arbeitslösungen oder im Umfeld der immunologischen Reaktion selbst zu Instabilitäten und Denaturierungen. Diese führen dazu, dass sowohl die Tertiär- als auch die Quartärstruktur des Proteins in einer Weise verändert wird, dass der Einsatzstoff nicht mehr für den Einsatz im Immunoassay verwendet werden kann.

Unter ungünstigen Bedingungen trennen sich die zusammengehörenden Untereinheiten der als spezifische Bindepartner eingesetzten Proteine voneinander. Diese Dissoziation von Untereinheiten kann im Falle von natürlicherweise kovalenten Verbrückungen beispielsweise durch Reduktion von Disulfidbrücken durch übliche Pufferzusätze wie DTT verursacht werden.

Noch größer ist allerdings die Gefahr der Dissoziation von nicht kovalent verbundenen Untereinheiten eines Proteins, die über Ladungen oder hydrophobe Wechselwirkungen zusammengehalten werden. Bei solchen Proteinen kann die Dissoziation der Untereinheiten bereits sehr leicht durch gängige Pufferzusätze wie Salze oder Detergenzien oder ungünstige pH- und Temperaturschwankungen ausgelöst werden. Eine einzelne, quasi ungeschützte Untereinheit ist damit auch der Denaturierung ausgesetzt. Dadurch kann die Tertiärstruktur des Proteins bzw. der einzelnen Untereinheit stark verändert werden. Dies bedeutet, dass auch die immunologischen Eigenschaften wie beispielsweise die Zugänglichkeit von wichtigen Epitopen so stark verändert werden, dass das als Bindepartner im Immunoassay eingesetzte Protein immunologisch nicht mehr erkannt, das heißt nicht mehr spezifisch gebunden wird.

Eine weitere Gefahr der Dissoziation von Untereinheiten besteht darin, dass aufgrund der Einstellung des chemischen Gleichgewichts mit verschiedenen Markierungsgruppen versehene Untereinheiten wieder reassoziieren. Wird im konkreten Fall ein aus zwei Untereinheiten bestehendes Protein für den Einsatz in einem Antikörper-Nachweis im Brückentestformat einerseits für den Einsatz an einer universellen Festphase derivatisiert, andererseits das gleiche Protein aber auch als signalgebende Komponente eingesetzt und für diesen Zweck an einen Marker (z.B. ein Enzym, Fluoreszenz- oder Chemilumineszenzmarker) gekoppelt, so kann folgendes geschehen: Eine anfänglich mit Positivproben (Proben, die den Analyten enthalten) erzeugte Eichkurve wird über den Zeitverlauf zunehmend flacher. Die Signale für negative Proben (Leerwerte) steigen und nähern sich den Werten für die oberen Positivproben mehr und mehr an, so dass keine Differenzierung mehr zwischen analytfreien und analythaltigen Proben mehr möglich ist.

In der deutschen Patentanmeldung DE 26 15 349 wird ein Verfahren zur chemischen Modifikation von Enzymen durch Umsetzung mit Chinonen beschrieben. Diese Modifikationen bewirken einen Stabilitätsgewinn, der eine verbesserte Enzymaktivität zur Folge hat. Es wird darauf hingewiesen, dass die Vernetzung der Enzymmoleküle untereinander, das heißt intermolekular, aber auch intramolekular erfolgen kann. Der Erhalt immunreaktiver Epitope spielt hierbei keine Rolle. Der Einsatz der mit Chinonen modifizierten Enzyme in immundiagnostischen Verfahren wird nicht beschrieben.

Debyser und De Clercq (Protein Science 1996, 5, S. 278-286) beschreiben die Vernetzung der beiden Untereinheiten der HIV-1 Reversen Transkriptase (RT) mittels Dimethylsuberimidat, wodurch Lysin-Seitenketten miteinander verknüpft werden. Zweck der Vernetzung ist die Untersuchung der Dimerisierung der beiden RT-Untereinheiten. Nur die dimere RT ist enzymatisch aktiv. Die kovalente Vernetzung der beiden Untereinheiten findet in Gegenwart verschiedener Inhibitoren statt. Je nach Effektivität des Inhibitors sind nach der chemischen Vernetzungsreaktion mehr oder weniger stark vernetzte RT-Moleküle bzw. Multimere entstanden. Die Auswirkung der Vernetzung auf immunologisch relevante Epitope oder die Verwendung der vernetzten Moleküle in Immunoassays spielt keine Rolle.

In der EP-A-0 331 068 wird die Verwendung von intermolekular vemetzten Immunglobulinen in Immunoassays offenbart. Das heißt mehrere Immunglobulin-Moleküle bzw. deren Fragmente werden miteinander kovalent verknüpft. Die Multimere von Antikörpern bzw. ihren Fragmenten werden als Entstörreagenz eingesetzt. Die vemetzten Immunglobuline bzw. ihre Fragmente sollen auf Immunglobuline gerichtete Störfaktoren in Humanseren beseitigen.

Die im Stand der Technik beschriebenen vemetzten Proteine, die unter natürlichen Bedingungen aus mehreren Untereinheiten bestehen, sind für den Einsatz als Antigene bzw. immunologische Bindepartner nicht oder nur bedingt geeignet, da im allgemeinen intermolekulare Multimere aus mehreren Proteinmolekülen entstehen. Diese Multimere sind für den Einsatz in Immunoassays nur bedingt geeignet, da ihre Größe zumeist nicht genau definiert ist. Die Multimere existieren somit lediglich in statistischer Größenverteilung, das heißt es gibt Mono,- Di, Tri-, Tetramere etc nebeneinander in einem Ansatz. Durch die undefinierte Vernetzung kann es zur Verdeckung von Epitopen kommen. Dies kann dazu führen, dass beispielsweise ein nachzuweisender Proben-Antikörper nicht an die verdeckten Epitope des Antigens binden kann und somit ein falsch negatives Ergebnis erhalten wird.

Ein weiteres Problem bei der Verwendung von Multimeren als immunologische Bindepartner ist die Tatsache, dass das Risiko unspezifischer Bindung von Störfaktoren an die multimeren Proteine steigt, die in der Probe vorhanden sind. Störfaktoren wie beispielsweise Rheumafaktoren haben oftmals mehrere schwach affine Bindungsstellen. Werden nun multimere Proteine als immunologische Bindepartner eingesetzt, kann dies dazu führen, dass insbesondere die Störfaktoren viele Angriffspunkte, das heißt Bindungsstellen bei den multimeren Proteinen finden. Als Folge davon können falsch-positive Testergebnisse erhalten werden, und die Spezifität des Immunoassays wird insgesamt stark verringert. Aufgabe war es daher, hinsichtlich ihrer Stabilität verbesserte Proteine bereitzustellen, die in Immunoassays als Bindepartner eingesetzt werden können. Die so verbesserten Proteine sollten eine gute Epitopzugänglichkeit besitzen, und es sollte die Spezifität des immunologischen Nachweisverfahren, in dem die Proteine eingesetzt werden, gewahrt bleiben.

Die Aufgabe wird gelöst durch die in den unabhängigen Ansprüchen beschriebene Erfindung. Die abhängigen Ansprüche stellen bevorzugte Ausführungsformen dar.

Es hat sich überraschenderweise gezeigt, dass sich praktisch ausschließlich intramolekular vernetzte Reverse Transkriptase von HIV ohne Verlust ihrer immunologischen Eigenschaften herstellen lasst und diese in vorteilhafter Weise in immunologischen Testverfahren als immunologischer Bindepartner eingesetzt werden Kann. Die ohne Vernetzung auftretenden Stabilitätsprobleme der Proteine werden somit weitgehend verhindert. Die Erfindung betrifft daher die Verwendung intramolekular kovalent vernetzter Reverse Transkriptase von HIV als immunologische Bindepartner in immunologischen Testverfahren.

Reverse Transkriptase von HIV neigt aufgrund ihrer Faltung, das heißt ihrer Tertiär- oder Quartärstruktur dazu, unter den Bedingungen eines Immunoassays ihre Faltung zu verlieren, zu denaturieren oder in ihre verschiedenen Untereinheiten zu dissoziieren. Bei einer solchen strukturellen Änderung besteht die Gefahr, dass immunologisch wichtige Epitope so verändert werden, dass sie beispielsweise von Antikörpern nicht mehr spezifisch gebunden werden. Im schlimmsten Fall bedeutet dies, dass ein immunologischer Test negativ ausfällt, das heißt die Anwesenheit des nachzuweisenden Antikörpers nicht anzeigt, weil die als Bindepartner eingesetzten Proteine denaturiert sind. Diese Nachteile werden durch den erfindungsgemä-βen Einsatz der intramolekular kovalent vemetzten Proteine weitgehend vermieden.

Erfindungsgemäß wird, Reverse Transkriptase von HIV, ganz besonders bevorzugt Reverse Transkriptase von HIV-1 eingesetzt.

Die Herkunft der reversen Transkriptase kann beliebiger Natur sein. Die zu vernetzenden Proteine können aus ihrer natürlichen Quelle, also beispielsweise einem Organismus oder Virus isoliert werden. Bevorzugt ist jedoch die Verwendung mittels gentechnologischer Methoden rekombinant hergestellter Proteine. Ganz besonders bevorzugt wird eine rekombinant hergestellte aufgereinigte RT verwendet, die von einem Expressionsklon wie er beispielsweise bei Müller et al. in J. Biol. Chem. 264/24: 13975-13978 (1989) beschrieben ist, exprimiert wird.

Bei den intramolekular kovalent vernetzten Proteinen ist es wichtig, dass die für die immunologische Erkennung entscheidenden Epitope durch die Vernetzung nicht oder nur so geringfügig verändert werden, dass der andere immunologische Bindepartner im Test das vernetzte Protein genauso gut erkennt und spezifisch bindet wie das unvernetzte Protein. Durch die Vernetzung dürfen also keine immunologisch relevanten Artefakte erzeugt werden, die das Testergebnis verfälschen können.

Unter der Bezeichnung Protein werden alle Polypeptide verstanden, die aus mindestens etwa 50 Aminosäuren, bevorzugt aus mindestens 100 Aminosäuren bestehen. Modifizierte Proteine, die beispielsweise mit Zuckerresten, Sialinsäuren oder Lipidstrukturen verknüpft sind, werden ebenfalls unter dem Begriff Protein verstanden.

Unter dem Begriff "intramolekular kovalent vernetzt" werden Proteine verstanden, deren Polypeptidkette mittels chemischer Modifikation so miteinander verknüpft ist, dass diese nicht mehr auseinanderfallen kann, das heißt ihre Tertiärstruktur und damit die Zugänglichkeit wichtiger Epitope nicht verliert. Im Falle eines Proteins, das aus mehrerem Untereinheiten besteht, bewirkt die intramolekulare kovalente Vernetzung, dass sowohl die Tertiär- als auch die Quartärstruktur erhalten bleibt. Die verschiedenen Polypeptidketten können aufgrund der Modifikation nicht mehr auseinanderdiffundieren.

Wesentlich ist, dass die kovalente Verknüpfung nur innerhalb eines Proteinmoleküls erfolgt.

Bei reverser Transkriptase, die aus mehreren Untereinheiten besteht, erfolgt die Verknüpfung erfindungsgemäß nur zwischen denjenigen Untereinheiten, die auch natürlicherweise ein intaktes Proteinmolekül bilden. Das heißt die Größe bzw. das Molekulargewicht des erfindungsgemäß intramolekular kovalent vemetzten Proteins werden nur geringfügig durch die vernetzende chemische Substanz erhöht. Praktisch ausgeschlossen sind Verknüpfungen, die innerhalb mehrerer Proteinmoleküle erfolgen, so dass sich Oligo- oder gar Polymere der Proteine bilden.

Erfindungsgemäß können die vemetzten Proteine vor oder nach der Vernetzung mit weiteren Modifikationsgruppen versehen werden, die beispielsweise für den Einsatz als markierte Antigene oder für die Bindung der vernetzten Proteine an eine Festphase erforderlich sind. Beispielhaft seien hier die Verknüpfung mit Biotin oder Streptavidin oder mit signalgebenden Markierungsgruppen wie Enzymen, Fluoreszenz- oder Chemilumineszenzgruppen erwähnt. Dem Fachmann sind solche Modifikationen geläufig. Durch diese Modifikationen dürfen sich die immunologischen Eigenschaften der erfindungsgemäß intramolekular vernetzten Proteine nicht oder nur insoweit verändern, dass eine Erkennung durch die spezifischen Bindepartner im Immunoassay immer noch gewährleistet ist.

Die nahezu ausschließlich intramolekulare Verknüpfung der Proteine kann insbesondere für Proteine mit Quartärstruktur beispielsweise mittels SDS-Polyacrylamid-Gelelektrophorese (SDS-PAGE) mit anschließender Coomassie-Blau-Färbung nachgewiesen werden. Es sollen nach der erfindungsgemäßen Vernetzung des Proteins in einem SDS-PAGE-Gel mit dem bloßen Auge keine größeren Molekulargewichte erkennbar sein als dem natürlichen Molekulargewicht des Proteins entspricht. Wird beispielsweise ein miniaturisiertes, kommerziell erhältliches SDS-Polyacrylamid-Gradientengel von 8 bis 25 % Polyacrylamid des Phast™-Systems der Firma Pharmacia eingesetzt, beträgt die aufgetragene Proteinmenge pro Spur etwa 500 ng. Bei dieser Menge lassen sich in diesem System erfindungsgemäß mit dem bloßen Auge keine größeren Molekulargewichte erkennen als dem natürlichen Molekulargewicht entspricht. Bei Proteinen, die natürlicherweise mehrere Untereinheiten, das heißt mehrere Polypeptidketten besitzen, darf das Molekulargewicht einer Bande nach der Vernetzung nicht die Summe der Molekulargewichte der Untereinheiten übersteigen. Proteinbanden auf dem Gel, die ein Molekulargewicht aufweisen, welches der Summe des Molekulargewichts der Untereinheiten entspricht, können als Nachweis für eine erfolgreiche intramolekulare Vernetzung eines Proteins mit Quartärstruktur angesehen werden. Mittels SDS-PAGE kann also einerseits die erfolgreiche intramolekulare Vernetzung eines aus mehreren Untereinheiten bestehenden Proteins und andererseits die Abwesenheit von Multimeren nachgewiesen werden.

Eine weitere Möglichkeit, die Abwesenheit von Multimeren nachzuweisen, bietet die Gelpermeationschromatographie, auch Gelausschluss-Chromatographie genannt, die beispielsweise mittels einer handelsüblichen HPLC-Apparatur durchgeführt werden kann. Proteinkomplexe, die ein Molekulargewicht aufweisen, das einem Multimeren des einzelnen Proteins entspricht, werden deutlich früher eluiert als einzeln vorliegende Proteine. Solche Multimere dürfen erfindungsgemäß nur zu einem niedrigen Prozentsatz vorliegen. Gemessen am Integral eines HPLC-Chromatogramms bedeutet dies, dass die Multimere nur bis zu einem Anteil von bis zu ca. 5 Prozent gemessen am eluierten Peak (Integral) des erfindungsgemäß nur intramolekular vernetzten Proteins betragen dürfen.

Als immunologische Bindepartner werden alle Moleküle bezeichnet, die unter den Bedingungen eines Immunoassays an andere Moleküle spezifisch binden können. Insbesondere sollen immunologische Bindepartner spezifisch den Analyten oder eine an den Analyten gebundene Substanz spezifisch binden können. Eine klassische Konstellation ist die spezifische Bindung eines Antikörpers an ein Antigen, beispielsweise die Bindung eines Anti-PSA-Antikörpers an PSA. Sowohl Antikörper als auch Antigen stellen immunologische Bindepartner dar. Erfindungsgemäß wird die intramolekular kovalent vernetzte (Reverse Transkriptase von HIV) als immunologischer Bindepartner in Immunoassays eingesetzt. Bevorzugt werden Antigene als immunologischen Bindepartner eingesetzt, wenn der Nachweis eines gegen diese Antigene gerichteten Antikörpers erfolgen soll. Bevorzugt sei hier der in einem unteren Abschnitt erläuterte Nachweis von Anti-HIV-RT-Antikörpern mittels erfindungsgemäß vernetzter HIV Reverser Transkriptase genannt.

Gegenstand der Erfindung ist auch ein immunologisches Testverfahren zum Nachweis eines Analyten in einer Probe. Das Verfahren ist dadurch gekennzeichnet, dass als immunologischer Bindepartner intramolekular kovalent vernetzte (Reverse Transkriptase von HIV) einesetzt wird. Es hat sich gezeigt, dass intramolekular kovalent vernetzte Proteine, insbesondere solche, die natürlicherweise aus mehreren Untereinheiten bestehen, unter den Bedingungen eines Immunoassays deutlich stabiler sind als unvernetzte Proteine.

Die verschiedenen Formate und Ausführungsformen von Immunoassays sowie die verschiedenen Nachweisverfahren wie beispielsweise über enzymatische Reaktionen, fluoreszierende oder chemilumineszierende Substanzen sind dem Fachmann geläufig und brauchen daher hier nicht gesondert erläutert zu werden. Erfindungsgemäß wird ein heterogenes Testformat, bei dem nach erfolgter immunologischer Reaktion die Abtrennung der festen von der flüssigen Phase erfolgt, gewählt.

Bevorzugt handelt es sich bei dem Verfahren um einen Immunoassay zur Diagnose von HIV-Infektionen. Liegt bei einem Patienten eine HIV-Infektion vor, so kann diese anhand der gegen bestimmte Antigene des Virus gebildeten Antikörper in einer Blut-, Serum- oder Plasmaprobe nachgewiesen werden. Häufig lassen sich auch die Virus-Antigene des HIV selbst nachweisen wie beispielsweise das p24-Antigen von HIV-1. Hierzu ist der Einsatz spezifischer, gegen das HIV-Antigen - hier: gegen p24 gerichteter Antikörper erforderlich.

Oftmals findet der Nachweis einer HIV-Infektion in einer Probe als kombinierter Antigen- und Antikörper-Nachweis-Test statt. Solche Tests werden auch als Kombitests bezeichnet. In der WO 98/40744 wird ein solcher Kombitest offenbart. Hier werden sowohl HIV-Antigene, das heißt p24-Antigen von HIV-1 bzw. HIV-1 Subtyp O und das entsprechende p26-Antigen von HIV-2 mittels spezifischer Antikörper detektiert als auch gegen HIV gerichtete Antikörper gegen Hüllproteine (env) des Erregers wie gp160, gp120, gp41 von HIV-1 und gp140, gp110, gp36 von HIV-2. Weiterhin werden in dem Kombitest gemäß WO 98/40744 auch Antikörper gegen HIV-RT nachgewiesen. Als immunologischer Bindepartner wird hierzu rekombinant hergestellte HIV-1 Reverse Transkriptase eingesetzt, die jedoch nicht intramolekular kovalent verknüpft ist.

Erfindungsgemäß wird intramolekular kovalent verknüpfte RT von HIV, insbesondere RT von HIV-1 bevorzugt in einem Kombitest zum Nachweis einer HIV-Infektion in einer Probe eingesetzt.

Ebenfalls ein Gegenstand der Erfindung ist intramolekular kovalent vernetzte Reverse Transkriptase von HIV, ein Enzym, das natürlicherweise in zwei Untereinheiten vorliegt. Die HIV RT liegt unter natürlichen Bedingungen als Heterodimer vor. HIV-1 RT besteht aus einer Untereinheit von 51 kDa und einer Untereinheit von 66 kDa. Die rekombinante Form ist beispielsweise über Expressionsklone verfügbar (beispielsweise von Müller et al. 1989 in J. Biol. Chem. 264/24, S. 13975-13978). Aufgrund eines Homologiegrades von etwa 60 %, abschnittweise sogar 100 % auf Aminosäureebene kann die HIV-1 RT im allgemeinen dazu verwendet werden, auch gegen HIV-2 RT gerichtete Antikörper zu erkennen. Die Bezeichnung HIV beinhaltet HIV-1, HIV-2, sowie alle Subtypen und Untergruppierungen des Virus wie beispielsweise den HIV-1 Subtyp O. Bevorzugt wird HIV-1 RT in intramolekular kovalent vernetzter Form.

Es hat sich überraschender Weise gezeigt, dass durch die intramolekulare kovalente Vernetzung HIV RT unter den Bedingungen des Immunoassays deutlich stabiler ist als in unvemetzter Form. Auch Temperaturbelastungen, wie sie beispielsweise bei längerer oder unsachgemäßer Lagerung oder auch unter Assaybedingungen ausgesetzt ist, hält die erfindungsgemäße RT deutlich besser stand als in unvernetzter Form. Die erfindungsgemäße intramolekular kovalent vernetzte RT zeichnet sich dadurch aus, dass die beiden Untereinheiten miteinander kovalent verknüpft sind, wobei keine intermolekularen Verknüpfungen mehrerer Moleküle vorliegen. Der Nachweis, dass keine Oligomere mehrerer RT-Moleküle vorliegen, kann beispielsweise mit der bereits erläuterten Gelausschluss-Chromatographie oder mittels SDS-PAGE erfolgen.

Als Vernetzungsreagenzien werden bevorzugt homo- und heterobifunktionelle Linker eingesetzt. Insbesondere werden bevorzugt MHS (3-Maleimidobenzoyl-N-hydroxysuccinimidester), EDC (1-Ethyl-3-(3-Dimethylaminopropyl)carbodiimid)), DSS (Disuccinimidylsuberat), HSAB (N-Hydroxysuccinimidyl-4-Azidobenzoat)), Sulfo-SANPAH (Sulfo-succinimidyl-6(4'-Amido-2'-Nitrophenylamido)hexanoat) zur intramolekularen Vernetzung der RT eingesetzt. Wie bereits erläutert, ist es wichtig, dass durch die chemische Reaktion der vernetzenden Linker nur eine intramolekulare Vernetzung des Proteins bzw. der beiden RT-Untereinheiten, nicht aber eine Vernetzung mehrerer RT-Moleküle untereinander erfolgt. Weiterhin wichtig ist es, dass durch die chemische Reaktion keine immunologisch relevanten Epitopen zerstört werden.

Ebenfalls ein Gegenstand der Erfindung ist ein Verfahren zur Herstellung intramolekular vernetzter Reverser Transkriptase von HIV. Das Verfahren umfasst die Schritte
- Bereitstellen der RT in gelöster Form
- gegebenenfalls Umsetzen der RT mit einem Blockierungsreagenz für SH-Gruppen
- Dialyse des Ansatzes gegen wässrigen Puffer
- Umsetzen der aktivierten RT mit einem der Vernetzungsreagenzien MHS (3-Maleimidobenzoyl-N-hydroxysuccinimidester), EDC (1-Ethyl-3-(3-Dimethylaminopropyl)carbodiimid)), DSS (Disuccinimidylsuberat), HSAB (N-Hydroxysuccinimidyl-4-Azidobenzoat)), Sulfo-SANPAH (Sulfosuccinimidyl-6(4'-Amido-2'-Nitrophenylamido)hexanoat)
- gegebenenfalls Stoppen der Reaktion
- Abtrennen der überschüssigen Reaktanten vom Reaktionsprodukt durch Dialyse
- gegebenenfalls Belichten des dialysierten Reaktionsprodukts mit UV-Licht.

Die bevorzugte Stöchiometrie von RT zu Vernetzungsreagenz beträgt etwa 1:1 bis 1:20. Die Verhältnisse der Reaktanten werden so gewählt, dass keine oder nur eine vernachlässigbar geringe Oligomerisierung mehrerer RT-Moleküle untereinander stattfindet.

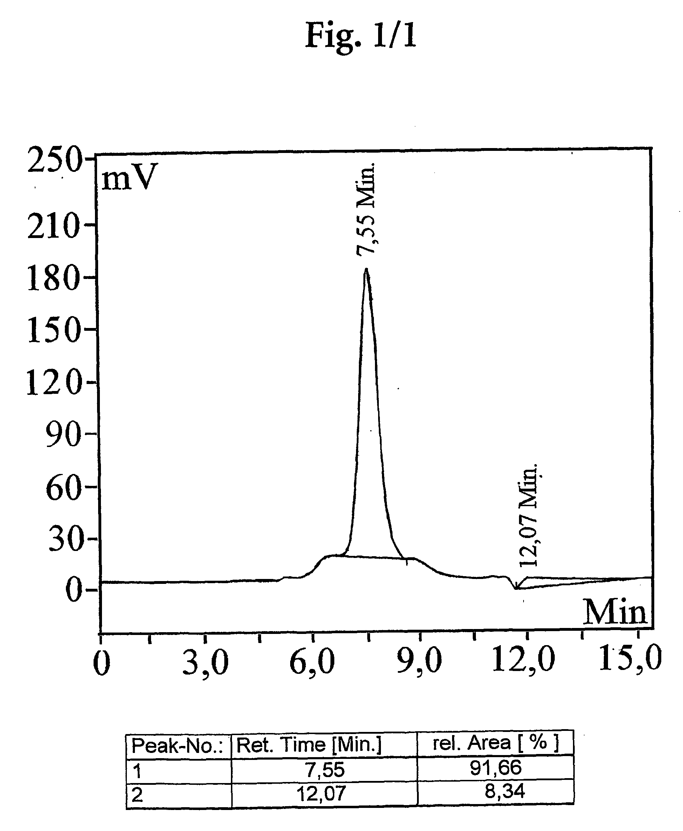

Figur 1 zeigt die Analyse der nach der Vernetzung erhaltenen Molekulargewichte der RT mittels Gelpermeationschromatographie.

Die folgenden Beispiele erläutern die Erfindung weiter.

### Beispiel 1

### Intramolekulare Vernetzung von HIV-1 Reverser Transkriptase

### a) Vernetzung mit MHS

HIV-1 Reverse Transkriptase (10 mg/ml) liegt gelöst vor in 50 mM Diethanolamin, pH 8,8, 25 mM NaCl, 1 mM DTT, 1 mM EDTA. Der pH-Wert wird durch Zugabe einer 1 M KH₂PO₄-Lösung auf pH 6,4 eingestellt

Durch Zugabe eines entsprechenden Aliquots einer 1 M Lösung NMM (N-Methylmaleinimid) in DMSO wird der Ansatz ad 5 mM NMM aufgestockt und anschließend für 60 min bei 25°C unter Rühren inkubiert. Anschließend erfolgt eine Dialyse gegen 50 mM Diethanolamin, pH 8,8, 25 mM NaCl.

Der pH-Wert wird nun durch Zugabe einer 1 M KH₂PO₄-Lösung auf pH 7.0 korrigiert. Vom MHS (3-Maleimidobenzoyl-N-hydroxysuccinimidester) wird eine Stammlösung in DMSO hergestellt (5 mg/ml). Die einer Angebotsstöchiometrie von 1:8 (mol Reverse Transkriptase / mol MHS) entsprechende Menge dieser Lösung wird zum Ansatz hinzugegeben, dieser dann wiederum für 60 min bei 25 °C unter Rühren inkubiert. Die Reaktion wird abgestoppt durch Aufstocken des Reaktionsgemisches ad 10 mM Lysin unter einer weiteren Inkubation für 30 min. Die Abtrennung überschüssiger Reaktanten erfolgt durch Dialyse gegen 10 mM Kaliumphosphatpuffer, pH 6,0, 50 mM NaCl, 1 mM EDTA.

Nach der Dialyse wird der pH-Wert durch Zugabe eines Aliquots einer 1 M K₂HPO₄-Lösung auf pH 7,4 eingestellt., der Ansatz wird für 4 h bei 25 °C unter Rühren weiterinkubiert, bevor Cystein ad 2 mM zugeben wird. Nach weiteren 30 min Inkubation wird die Reaktion durch Zugabe von NMM (ad 5 mM) abgestoppt. Der Ansatz wird dialysiert gegen 50 mM Diethanolamin, pH 8,8, 25 mM NaCl.

### b) Vernetzung mit EDC

HIV-1 Reverse Transkriptase (10 mg/ml) liegt gelöst vor in 50 mM Diethanolamin, pH 8,8, 25 mM NaCl, 1 mM DTT, 1 mM EDTA. Der pH-Wert wird durch Zugabe einer 1 M KH₂PO₄-Lösung auf pH 6,4 eingestellt

Durch Zugabe eines entsprechenden Aliquots einer 1 M Lösung NMM (N-Methylmaleinimid) in DMSO wird der Ansatz ad 5 mM NMM aufgestockt und anschließend für 60 min bei 25°C unter Rühren inkubiert. Anschließend erfolgt eine Dialyse gegen 10 mM Kaliumphosphatpuffer, pH 7,0, 50 mM NaCl.

Vom EDC (1-Ethyl-3-(3-Dimethylaminopropyl)carbodümid)) wird eine Stammlösung in DMSO hergestellt (2 mg/ml). Die einer Angebotsstöchiometrie von 1:10 (mol Reverse Transkriptase / mol EDC) entsprechende Menge dieser Lösung wird zum Ansatz hinzugegeben, dieser dann wiederum für 60 min bei 25 °C unter Rühren inkubiert. Die Abtrennung überschüssiger Reaktanten erfolgt durch Dialyse gegen 25 mM Kaliumphosphatpuffer, pH 7,0, 50 mM NaCl.

### c) Vernetzung mit DSS

HIV-1 Reverse Transkriptase (10 mg/ml) liegt gelöst vor in 50 mM Diethanolamin, pH 8,8, 25 mM NaCl, 1 mM DTT, 1 mM EDTA. Der pH-Wert wird durch Zugabe einer 1 M KH₂PO₄-Lösung auf pH 6,4 eingestellt.

Durch Zugabe eines entsprechenden Aliquots einer 1 M Lösung NMM (N-Methylmaleinimid) in DMSO wird der Ansatz ad 5 mM NMM aufgestockt und anschließend für 60 min bei 25 °C unter Rühren inkubiert. Anschließend erfolgt eine Dialyse gegen 10 mM Kaliumphosphatpuffer, pH 8,0, 25 mM NaCl.

Vom DSS (Disuccinimidylsuberat) wird eine Stammlösung in DMSO hergestellt (2 mg/ml). Die einer Angebotsstöchiometrie von 1:10 (mol Rev. Transkriptase / mol DSS) entsprechende Menge dieser Lösung wird zum Ansatz hinzugegeben, dieser dann wiederum für 60 min bei 25°C unter Rühren inkubiert. Die Abtrennung überschüssiger Reaktanten erfolgt durch Dialyse gegen 25 mM Kaliumphosphatpuffer, pH 7,0, 50 mM NaCl.

### d) Vernetzung mit HSAB

HIV-1 Reverse Transkriptase (10 mg/ml) liegt gelöst vor in 50 mM Diethanolamin, pH 8,8, 25 mM NaCl, 1 mM DTT, 1 mM EDTA. Der pH-Wert wird durch Zugabe einer 1 M KH₂PO₄-Lösung auf pH 6,4 eingestellt.

Durch Zugabe eines entsprechenden Aliquots einer 1 M Lösung NMM (N-Methylmaleinimid) in DMSO wird der Ansatz ad 5 mM NMM aufgestockt und anschließend für 60 min bei 25 °C unter Rühren inkubiert. Anschließend erfolgt eine Dialyse gegen 10 mM Kaliumphösphatpuffer, pH 8,0, 25 mM NaCl.

Vom HSAB (N-Hydroxysuccinimidyl-4-Azidobenzoat)) wird eine Stammlösung in DMSO hergestellt (2 mg/ml). Die einer Angebotsstöchiometrie von 1:5 (mol Rev. Transkriptase / mol HSAB) entsprechende Menge dieser Lösung wird zum Ansatz hinzugegeben, dieser dann wiederum für 60 min bei 25 °C unter Rühren inkubiert. Die Abtrennung überschüssiger Reaktanten erfolgt durch Dialyse gegen 25 mM Kaliumphosphatpuffer, pH 7,0, 50 mM NaCl.

Der Ansatz wird anschließend für 7 min mit einer UV-Lampe belichtet.

### e) Vernetzung mit Sulfo-SANPAH

HIV-1 Reverse Transkriptase (10 mg/ml) liegt gelöst vor in 50 mM Diethanolamin, pH 8,8, 25 mM NaCl, 1 mM DTT, 1 mM EDTA. Der pH-Wert wird durch Zugabe einer 1 M KH₂PO₄-Lösung auf pH 6,4 eingestellt.

Durch Zugabe eines entsprechenden Aliquots einer 1 M Lösung NMM (N-Methylmaleinimid) in DMSO wird der Ansatz ad 5 mM NMM aufgestockt und anschließend für 60 min bei 25 °C unter Rühren inkubiert. Anschließend erfolgt eine Dialyse gegen 10 mM Kaliumphosphatpuffer, pH 8,0, 25 mM NaCl.

Vom Sulfo-SANPAH (Sulfosuccinimidyl-6(4'-Amido-2'-Nitrophenylamido)hexanoat) wird eine Stammlösung in DMSO hergestellt (4 mg/ml). Die einer Angebotsstöchiometrie von 1:5 (mol Rev. Transkriptase / mol Sulfo-SANPAH) entsprechende Menge dieser Lösung wird zum Ansatz hinzugegeben, dieser dann wiederum für 60 min bei 25 °C unter Rühren inkubiert. Die Abtrennung überschüssiger Reaktanten erfolgt durch Dialyse gegen 25 mM Kaliumphosphatpuffer, pH 7,0, 50 mM NaCl.

Der Ansatz wird anschließend für 7 min mit einer UV-Lampe belichtet.

### Beispiel 2

### Nachweis der ausschließlich intramolekularen Vernetzung von HIV-1 Reverser Transkriptase

### a) SDS-Gelelektrophorese

Aliquots der intramolekular vemetzten HIV-1 Reversen Transkriptase wurden mittels Polyacrylamid-Gelelektrophorese in Gegenwart von SDS an einer Phast-Gel-Apparatur (Fa. Pharmacia™) nach einer Standardvorschrift des Herstellers analysiert.

Die nicht vernetzte Kontrolle weist ausschließlich Banden mit Molekulargewichten von 66 kD und 51 kD auf, die den Untereinheiten der Reversen Transkriptase entsprechen. Intramolekular vernetzte Reverse Transkriptase lässt Banden mit Molekulargewichten von 110-120 kD erkennen, die eine erfolgreiche Vernetzung der Untereinheiten untereinander beweisen. Größere Proteinkomplexe sind nicht nachweisbar, d.h. mit den erfindungsgemäßen Vernetzungsverfahren kommt es nicht zu einer intermolekularen Verbrückung mehrerer Moleküle Reverser Transkriptase.

### b) Analytische Gelpermeationschromatographie

Ein Aliquot der intramolekular vernetzten HIV-1 Reversen Transkriptase wurden einer Gelpermeationschromatographie an einer TSK 3000 Säule (Toso Haas™) mittels einer handelsüblichen HPLC-Apparatur nach einer Standardvorschrift des Herstellers analysiert.

Die intramolekular vernetzte Reverse Transkriptase eluiert von der Säule mit einer Retentionszeit, die globulären Proteinen mit Molekulargewichten von 100-130 kD entspricht (hier 7,5 min). Größere Proteinkomplexe, die eine kürzere Retentionszeit im Chromatogramm aufweisen würden, sind nicht nachweisbar, d.h. mit den erfindungsgemäßen Vernetzungsverfahren kommt es nicht zu einer intermolekularen Verbrückung mehrerer Moleküle Reverser Transkriptase zu oligomeren oder polymeren Strukturen. Das Chromatogramm ist in Figur 1 dargestellt.

### Beispiel 3

### Derivatisierung von intramolekular vernetzter HIV-1 Reverser Transkriptase am Beispiel der Biotinmarkierung

Intramolekular vernetzte HIV-1 Reverse Transkriptase (siehe Beispiel 1) liegt in Diethanolamin oder Kaliumphosphatpuffer vor. Die unvernetzte RT als Vergleich wird mit N-Methylmaleimid behandelt und gegen Diethanolamin dialysiert. Sofern erforderlich wird in allen RT-Ansätzen durch Zugabe von NaOH ein pH-Wert von pH 8,6 - 8,8 eingestellt. Vom Biotin-DDS (Biotinyl-diaminodioxaoctan-disuccinimidylsuberat) wird eine Stammlösung in DMSO hergestellt (6 mg/ml). Die einer Angebotsstöchiometrie von 1:4 (mol Rev. Transkriptase / mol Biotin-DDS) entsprechende Menge dieser Lösung wird zum Ansatz hinzugegeben, dieser dann wiederum für 60 min bei 25 °C unter Rühren inkubiert. Die Reaktion wird abgestoppt durch Aufstocken des Reaktionsgemisches ad 10 mM Lysin unter einer weiteren Inkubation für 30 min. Die Abtrennung überschüssiger Reaktanten erfolgt durch Dialyse gegen 50 mM Diethanolamin, pH 8.8, 25 mM NaCl.

### Beispiel 4

### Stabilitätsprüfing im Funktionstest

Zur Überprüfung der Stabilität der HIV-1 Reversen Transkriptase wurde ein Immunoassay am Elecsys® der Firma Roche Diagnostics GmbH, Mannheim durchgeführt. Vermessen wurden neben einer Negativkontrolle (NK), die keine Anti-RT-Antikörper enthält und einer Positivkontrolle (PK), die Anti-RT-Antikörper enthält, jeweils zwei HIV positive Humanseren mit Anti-RT-Reaktivität.

Dafür wurden 45 µl Probe zusammen mit 55µl Reagenz 1 (biotinylierte RT) und 55 µl Reagenz 2 (Ruthenium-markierte RT) für 9 min bei 37 °C inkubiert. Anschließend wurden Streptavidin-beschichtete Magnetbeads zugesetzt und die Mischung weitere 9 min inkubiert. Danach erfolgte ein magnetisches Capturing der Beads und die quantitative Erfassung des Elektrochemiluminiszenzsignals.

Um die Stabilität der biotinylierten Reversen Transkriptase in erfindungsgemäß vernetzter und in unvernetzter Form vergleichend zu überprüfen, wurde vor Durchführung des Tests das Reagenz 1 (biotinylierte RT) die unten angegebene Zeit 18 Stunden lang bei 42°C belastet. Als Bezugsgröße diente ein gleichzeitig angesetztes und bei 4 °C gelagertes Reagenz 1. Alle anderen Reagenzien wurden für die Versuche jeweils frisch angesetzt.

Die Auswertung erfolgt über die Signaldynamik, das heißt man ermittelt den Quotienten aus dem erhaltenen Signal und der jeweiligen Negativkontrolle. Je größer der Wert für die Signaldynamik ist, desto besser kann eine Differenzierung zwischen HIV-Antikörper-positiven und -negativen Proben erfolgen. Eine große Signaldynamik ist also erstrebenswert.

Für den Vergleich von belasteter RT zu unbelasteter RT wurden die jeweiligen Werte in Relation zueinander gesetzt. Die Ergebnisse sind in Tabelle 1 dargestellt

**Tabelle 1**

| | **rekombinante HIV-1-RT-Bi(DDS); nicht vernetzt** | | | | | |
|---|---|---|---|---|---|---|
| | unbelastet | | Belastung 18 h 42 °C | | Vergleich belastet/unbelastet | |
| Proben | Counts | Signaldynamik | Counts | Signaldynamik | Counts | Signaldynamik |
| Negativkontrolle | 1763 | **1,0** | 1049 | **1,0** | 60% | **100%** |
| Positivkontrolle | 16848 | **9,6** | 1480 | **1,4** | 9% | **15%** |
| HIV-Serum 1 | 6209 | **3,5** | 1054 | **1,0** | 17% | **29%** |
| HIV-Serum 2 | 5162 | **2,9** | 917 | **0,9** | 18% | **30%** |
| HIV-Serum 3 | 5832 | **3,3** | 1150 | **1,1** | 20% | **33%** |
| HIV-Serum 4 | 111444 | **63,2** | 6267 | **6,0** | 6% | **9%** |
| | | | | | | |
| | | | | | | |

| | **rekombinante HIV-1-RT(MHS)-Bi(DDS); erfindungsgemäβ vernetzt** | | | | | |
|---|---|---|---|---|---|---|
| | unbelastet | | Belastung 18 h 42 °C | | Vergleich belastet/unbelastet | |
| Proben | Counts | Signaldynamik | Counts | Signaldynamik | Counts | Signaldynamik |
| Negativkontrolle | 1304 | **1,0** | 1206 | **1,0** | 92% | **100%** |
| Positivkontrolle | 73335 | **56,2** | 77611 | **64,4** | 106% | **114%** |
| HIV-Serum 5 | 8476 | **6,5** | 7092 | **5,9** | 84% | **90%** |
| HIV-Serum 6 | 14504 | **11,1** | 10615 | **8,8** | 73% | **79%** |
| HIV-Serum 7 | 69459 | **53,3** | 59347 | **49,2** | 85% | **92%** |
| HIV-Serum 8 | 168674 | **129,4** | 168304 | **139,6** | 100% | **108%** |

Es zeigt sich, dass auch nach mehrstündiger Belastung bei einer erhöhten Temperatur im Vergleich zu unbelasteter RT die Signaldynamik im Immunoassay mit erfindungsgemäß vernetzter RT noch mindestens 79%, bevorzugt mindestens 90 % beträgt, während die Signaldynamik bezogen auf die Negativkontrolle bei unvernetzter RT nur bei höchstens etwa 30 % liegt, zum Teil bei deutlich unter 30 % oder sogar unter 20 %. Die Signale fallen bei Verwendung unvernetzter RT auf das Niveau von Negativseren ab, während die erfindungsgemäß vernetzte RT seine immunologische Funktion beibehält. Die von den Probenantikörpern erkannten Epitope der RT bleiben also trotz Temperaturbelastung weitgehend erhalten. Dies bedeutet, dass die erfindungsgemäß vernetzte RT deutlich stabiler ist als die unvernetzte.

## Patentansprüche

1. Verwendung mittels chemischer Modifikation Intramolekular kovalent vernetzter Virus-Proteine als Antigen beim Nachweis eines gegen dieses Antigen gerichteten Antikörpers in immunologischen Testverfahren im heterogenen Testformat, bei dem nach erfolgter immunologischer Reaktion die Abtrennung der festen von der flüssigen Phase erfolgt, **dadurch gekennzeichnet, dass** als Antigen Reverse Transkriptase von HIV eingesetzt wird.

2. immunologisches Testverfahren zum Nachweis eines Analyten in einer Probe, **dadurch gekennzeichnet, dass** als immunologischer Bindepartner ein intramolekular kovalent quervernetztes Antigen gemäß Anspruch 1 eingesetzt wird.

3. Immunologisches Testverfahren nach Anspruch 2 **dadurch gekennzeichnet, dass** es sich um ein verfahren zur Diagnose von HIV-Infektionen handelt.

4. Intramolekular kovalent vernetzte Reverse Transkriptase (RT) von HIV, bei der ausschließlich die beiden Untereinheiten der RT miteinander kovalent vernetzt sind, wobei keine intermolekularen Verknüpfungen mehrerer RT-Moleküle miteinander vorliegen, **dadurch gekennzeichnet, dass** als Vernetzungsreagenz MHS (3-Maleimidobenzoyl-N-hydroxysuccinimidester), EDC (1-Ethyl-3- (3-Dimethylaminopropyl)carbodiimid)), DSS (Disuccinimidylsuberat), HSAB (N-Hydroxy-succinimidyl-4-AXdobenzoat)), oder Sulfo-SANPAH (Sulfosuccinimidyl-6(4'-Amido-2'- Nitrophenylamido) hexanoat) eingesetzt wird.

5. Verfahren zur Herstellung intramolekular vernetzter Reverser Transkriptase von HIV gemäß Anspruch 4 umfassend die Schritte
(a) Bereitstellen der RT in gelöster Form
(b) Umsetzen der RT mit einem Blockierungsreagenz für SH-Gruppen
(c) Dialyse des Ansatzes gegen wässrigen Puffer
(d) Umsetzen der aktivierten RT mit einem der Vernetzungsreagenzien MHS (3-Maleimidobenzoyl-N-hydroxysuccinimidester), EDC (1-Ethyl-3- (3-Dimethylaminopropyl)carbodiimid)), DSS (Disuccinimidylsuberat), HSAB (N-Hydroxy-succinimidyl-4-Azidobenzoat)), Sulfo-SANPAH (Sulfosuccinimidyl-6(4'-Amido-2'-Nitrophenylamido) hexanoat), wobei eine Stöchiometrie von RT zu Vernetzungsreagenz von 1:1 bis 1:20 gewählt wird
(e) gegebenenfalls Stoppen der Reaktion
(f) Abtrennen der überschüssigen Reaktanten vom Reaktionsprodukt durch Dialyse
(g) gegebenenfalls Belichten des dialysterten Reaktionsprodukts mit UV-Licht.

## Claims

1. Use of virus proteins intramolecularly covalently cross-linked by means of chemical modification as an antigen for the detection of an antibody directed against this antigen in immunological test methods in a heterogeneous test format in which the solid phase is separated from the liquid phase after the immunological reaction is completed, **characterized in that** reverse transcriptase of HIV is used as the antigen.

2. Immunological test method for the detection of an analyte in a sample, **characterized in that** an intramolecularly covalently cross-linked antigen according to claim 1 is used as the immunological binding partner.

3. Immunological test method according to claim 2, **characterized in that** it is a method for diagnosing HIV infections.

4. Intramolecularly covalently cross-linked reverse transcriptase (RT) of HIV in which solely the two subunits of RT are covalently cross-linked to one another and no intermolecular linkages between several RT molecules are present, **characterized in that** MHS (3-maleimidobenzoyl-N-hydroxysuccinimide ester), EDC (1-ethyl-3-(3-dimethylaminopropyl)carbodiimide)), DSS (disuccinimidyl suberate), HSAB (N-hydroxy-succinimidyl-4-azidobenzoate)) or sulfo-SANPAH (sulfosuccinimidyl-6[4'-amido-2'-nitrophenylamido)hexanoate) is used as a cross-linking reagent.

5. Process for producing intramolecularly cross-linked reverse transcriptase of HIV according to claim 4 comprising the steps
(a) preparing the RT in a dissolved form
(b) reacting the RT with a blocking reagent for SH groups
(c) dialysing the preparation against aqueous buffer
(d) reacting the activated RT with one of the cross-linking reagents MHS (3-maleimidobenzyol-N-hydroxysuccinimide ester), EDC (1-ethyl-3-(3-dimethylaminopropyl)carbodiimide)), DSS (disuccinimidyl suberate), HSAB (N-hydroxy-succinimidyl-4-azidobenzoate)) or sulfo-SANPAH (sulfosuccinimidyl-6[4'-amido-2'-nitrophenylamido)hexanoate), wherein a stoichiometry of RT to cross-linking reagent of 1:1 to 1:20 is selected
(e) optionally stopping the reaction
(f) separating the excess reactants from the reaction product by dialysis
(g) optionally exposing the dialysed reaction product to UV light.

## Revendications

1. Utilisation de protéines virales réticulées par covalence de manière intramoléculaire par modification chimique comme antigène lors de la détection d'un anticorps dirigé contre cet antigène dans des procédés de test immunologiques dans un format de test hétérogène, dans lequel, après la réaction immunologique, on réalise une séparation de la phase solide de la phase liquide, **caractérisée en ce qu'**on utilise comme antigène une transcriptase inverse du VIH.

2. Procédé test immunologique destiné à la détection d'un analyte dans un échantillon, **caractérisé en ce qu'**on utilise comme partenaire de liaison immunologique un antigène réticulé par covalence de manière intramoléculaire selon la revendication 1.

3. Procédé de test immunologique selon la revendication 2, **caractérisé en ce qu'**il s'agit d'un procédé pour le diagnostic d'infections par le VIH.

4. Transcriptase inverse du VIH (reverse transcriptase - RT) réticulée par covalence de manière intramoléculaire, dans laquelle seules les deux sous-unités de la RT sont réticulées l'une avec l'autre par covalence, des liaisons intermoléculaires de plusieurs molécules de RT n'existant pas, **caractérisée en ce qu'**on utilise, comme réactif de réticulation, le MHS (ester de 3-maléimidobenzoyl-N-hydroxysuccinimide), l'EDC (1-éthyl-3-(3-diméthylaminopropyl)carbodiimide)), le DSS (subérate de disuccinimidyle), le HSAB (N-hydroxysuccinimidyl-4-azidobenzoate)), ou le sulfo-SANPAH (6(4'-amido-2'-nitrophénylamido)hexanoate de sulfosuccinimidyle).

5. Procédé pour la préparation de transcriptase inverse du VIH réticulée de manière intramoléculaire selon la revendication 4, comprenant les étapes
(a) mise à disposition de la RT sous forme dissoute
(b) transformation de la RT avec un réactif de blocage pour les groupes SH
(c) dialyse de la charge contre un tampon aqueux
(d) transformation de la RT activée avec un des réactifs de réticulation MHS (ester de 3-maléimidobenzoyl-N-hydroxysuccinimide), EDC (1-éthyl-3-(3-diméthylaminopropyl)carbodiimide)), DSS (subérate de disuccinimidyle), HSAB (N-hydroxysuccinimidyl-4-azidobenzoate)), sulfo-SANPAH (6(4'-amido-2'-nitrophénylamido)hexanoate de sulfosuccinimidyle), en choisissant une stoechiométrie de la RT au réactif de réticulation de 1:1 à 1:20
(e) le cas échéant arrêt de la réaction
(f) séparation des réactifs en excès du produit de réaction par dialyse
(g) le cas échéant irradiation du produit de réaction dialysé par de la lumière UV.
